# EUROPEAN PATENT SPECIFICATION

(11) **EP 3 696 169 B1**
(45) Date of publication and mention of the grant of the patent: **11.01.2023**
(21) Application number: 19170177.0
(22) Date of filing: 18.04.2019
(51) Int. Cl.: C07D 213/73, A61K 31/44, A61P 21/00

(54) **MANUFACTURING PROCESS FOR AMIFAMPRIDINE PHOSPHATE**
HERSTELLUNGSVERFAHREN FÜR AMIFAMPRIDINPHOSPHAT
PROCÉDÉ DE FABRICATION DE PHOSPHATE D'AMIFAMPRIDINE

(43) Date of publication of application: 19.08.2020
(73) Proprietor: Alfred E. Tiefenbacher (GmbH & Co. KG), 22767 Hamburg (DE)
(72) Inventor: Staver, Ruslan, 22767 Hamburg (DE); Dr. Michaelis, Simon, 14943 Luckenwalde (DE)
(74) Representative: Hamm&Wittkopp Patentanwälte PartmbB

(56) References cited:
- LE BAIL A ET AL: "Ab initio structure determination of 3,4-diaminopyridin-1-ium dihydrogen phosphate", POWDER DIFFRACTION, JCPDS-INTERNATIONAL CENTRE FOR DIFFRACTION DATA, WSARTHMORE, US, vol. 26, no. 4, 1 December 2011 (2011-12-01), pages 321-325, XP009515195, ISSN: 0885-7156, DOI: 10.1154/1.3660160 [retrieved on 2012-03-05]
- MAHE NATHALIE ET AL: "Crystal Structure and Solid-State Properties of 3,4-Diaminopyridine Dihydrogen Phosphate and Their Comparison with Other Diaminopyridine Salts", CRYSTAL GROWTH & DESIGN, ACS, WASHINGTON, DC, US, vol. 13, no. 2, 6 February 2013 (2013-02-06), pages 708-715, XP009515193, ISSN: 1528-7483, DOI: 10.1021/CG3014249 [retrieved on 2013-01-11]
- MANI RAJABOOPATHI ET AL: "Fluorescence Properties Reinforced by Proton Transfer in the Salt 2,6-Diaminopyridinium Dihydrogen Phosphate", JOURNAL OF PHYSICAL CHEMISTRY. A, MOLECULES, SPECTROSCOPY,KINETICS, ENVIRONMENT AND GENERAL THEORY, WASHINGTON, DC, US, vol. 118, no. 34, 28 August 2014 (2014-08-28), pages 6883-6892, XP009515190, ISSN: 1089-5639, DOI: 10.1021/JP504343A [retrieved on 2014-08-08]

## Description

The present invention relates to a process for the preparation of crystalline amifampridine phosphate.

Amifampridine (3,4-diaminopyridine, 3,4-DAP) has been widely used as an *ad-hoc* hospital preparation in the treatment of Lambert-Eaton myasthenic syndrome (LEMS). Since the on-demand preparations of amifampridine exhibited variability and a lack of reliability in the quality of the drug product, a tablet was developed containing 10 mg of amifampridine as a phosphate salt. This tablet is commercially available under the tradename Firdapse^{®} for the symptomatic treatment of LEMS. Firdapse^{®} is an immediate-release tablet containing microcrystalline cellulose, anhydrous colloidal silica and calcium stearate as pharmaceutical excipients, whereby the tablet is prepared by direct compression.

The preparation of amifampridine and its monohydrochloride salt is described in EP 0 159 112. US 2004/0106651 relates to the manufacture of the phosphate and tartrate salt of amifampridine and their use for the treatment of botulism, myasthenia, myasthenic syndromes and fatigue related to a neurological pathology, e.g. multiple sclerosis or amyotrophic lateral sclerosis (ALS). The crystal structure and solid state properties of amifampridine phosphate is described in Cryst. Growth Des. 2013, 13, 708-715. In this article, it is also mentioned that six amifampridine salts had been prepared (hydrogen chloride, hydrogen bromide, sulfate, dihydrogen phosphate, tartrate and benzoate), which are more stable than the free base, since the involvement of the lone electron pair on the pyridine nitrogen in the charge-assisted hydrogen bond reduces the possibility of oxidation for amifampridine. Among these salts, only two, namely the dihydrogen phosphate and hydrogen tartrate salt, have been selected for medical use because they appear physically and chemically stable in time, possess little hygroscopicity, have excellent solubility in water and have an acceptable pH in saturated solution.

The preparation and the crystalline structure of the dihydrochloride salt of amifampridine is disclosed in Acta Cryst. 2009, E65, o131, and in the supporting information. The salt was prepared by preparing a solution of amifampridine and HCl in ethanol and allowing the solution to slowly evaporate; the slow crystallization from ethanol gives needle-like crystals. As disclosed in EP 3 412 656, a number of crystalline forms of amifampridine dihydrochloride exist, which have an irregular shape. These forms may be obtained by combining an aqueous solution of the salt and an anti-solvent, e.g. acetone, i.e. by fast crystallization (precipitation).

US 2004/0106651 discloses a process for the manufacture of the phosphate salt of amifampridine, in which phosphoric acid (85 wt%) is slowly introduced into a solution of 3,4-DAP in water. The obtained precipitate was recrystallized from a mixture of ethanol and water for purification. The powder XRD-pattern and the single crystal XRD of the phosphate salt of amifampridine are disclosed in Powder Diffraction 2011, 26(4), 321-325, and Cryst. Growth Des. 2013, 13, 708-715. It was found that phosphoric acid is not strong enough to ionize a second nitrogen atom of one of the amino groups of 3,4-DAP, i.e. the phosphate salt of amifampridine crystallizes as dihydrogen phosphate (3,4-DAPH⁺ H₂PO₄⁻). Amifampridine dihydrogen phosphate is hereinafter referred to as amifampridine phosphate or phosphate salt of amifampridine (3,4-DAPP). As described in CrystEngComm 2014, 16, 2205-2219, 3,4-DAPP also crystallizes from a mixture of water and acetone.

It was found that the prior art methods give a voluminous, cotton-like material (needles partly aggregated) that cannot easily be processed into solid unit dosage forms. Thus, it was an object of the present invention to improve the processability of amifampridine phosphate. This object is solved by the subject matter as defined in the claims.

The amifampridine phosphate salt of the present invention is a crystalline material, whereby the crystals have an irregular shape or plate-like shape. These crystals do not show a cotton-like habit, and they may form aggregates. The salt of the present invention is a free-flowing material that can be easily filtered and processed into a solid unit dosage form without the need of micronization. Thus, the salt of the present invention is in particular suitable for preparing a solid unit dosage form, e.g. a tablet, through dry granulation or direct compression.

The phosphate salt of amifampridine of the present invention is obtainable by a process consisting of the steps:
i) preparing an alcoholic solution containing amifampridine, wherein the solvent is an alcohol optionally in admixture with not more than 10 wt% water, wherein the alcohol is selected from methanol, ethanol, n-propanol and iso-propanol,
ii) precipitating the phosphate salt of amifampridine by adding an aqueous phosphoric acid solution to the alcoholic solution obtained in step (i), wherein the aqueous phosphoric acid solution contains at least 70 wt% phosphoric acid, and
iii) isolating the phosphate salt of amifampridine obtained in step (ii).

The salt of the present invention is further characterized by the following peaks of its powder X-ray diffractogram (Cu-Kα1 = 1.54059 Å): 11.06, 12.16, 12.69, 12.91, 15.45, 17.12, 17.42, 18.07, 20.21, 21.60, 22.22, 22.57, 22.95, 24.03, 24.59, 25.11, 25.54, 26.05, 26.69, 27.02, 27.76, 28.21, 28.69, 29.54, 30.19, 30.27 and 30.44 ± 0.20° 2θ.

The crystallization of amifampridine phosphate from a solution in water as suggested in US 2004/0106651 gives an impure product that needs to be purified by recrystallization. The recrystallization from an ethanol/water mixture gave an off-white (beige), voluminous, cotton-like product. The relatively fast crystallization (precipitation) as used in the present invention, in which amifampridine phosphate is precipitated from an alcoholic solution by adding an aqueous phosphoric acid solution, on the other hand, gives a pure, free-flowing material that can be further processed without a purification step, such as recrystallization.

The crystalline form of amifampridine phosphate disclosed in the prior art, which is obtained by recrystallization of amifampridine phosphate from an ethanol/water mixture, is hereinafter designated as form 1. In the course of the inventor's investigation of the solid-state properties of amifampridine phosphate, one additional crystalline form has been detected, which is hereinafter designated as form 2.

### Terms:

All references to the "polymorph 1 form", "polymorphic form 1", "polymorph 1", "crystalline form 1", "API form 1" or "form 1" or the like may be used interchangeably for the characterization of the solid-state form of amifampridine phosphate API.

All references to the "polymorph 2 form", "polymorphic form 2", "polymorph 2", "crystalline form 2", "API form 2" or "form 2" or the like may be used interchangeably for the characterization of the solid-state form of amifampridine phosphate API.

The term "API" or "active pharmaceutically ingredient" herein refers to a pharmaceutically active molecule (e.g. amifampridine) as well as its pharmaceutically acceptable and therapeutically active salts (e.g. amifampridine phosphate), and polymorphs thereof, that induce a desired pharmacological or physiological effect. Terms like "active ingredient", "active drug ingredient", "active agent", or "drug substance" may be used synonymously for "API ".

The term "essentially the same" with reference to PXRD means that variabilities in peak positions and relative intensities of the peaks are to be taken into account. For example, a typical precision of the 2-Theta (2θ) values is in the range of ± 0.20° 2θ. Thus, for example a diffraction peak that usually appears at 17.40° 2θ for example can appear between 17.20° and 17.60° 2θ on most X-ray diffractometers under standard conditions. With respect to the relative intensities and the characteristic peaks of the powder X-ray diffraction patterns mentioned above, the provided values of relative intensity are not intended as limiting for the identification of the characteristic peaks mentioned. As is known to a skilled person, the relative peak intensities will show inter-apparatus variability, batch-to-batch variability, as well as variability due to degree of crystallinity, preferred orientation, sample preparation, and as such are provided as an indication and as qualitative measure only, but not a limiting definition, of the intensities of the peaks in the powder X-ray diffraction patterns.

### Figures:

- Figure 1:: XRPD of form 1
- Figure 2:: XRPD of form 1 in admixture with a small amount of form 2 (about 15%)
- Figure 3:: Microscopic image of form 1 obtained by the process of the present invention in silicone oil
- Figure 4:: Microscopic image of form 1 obtained by the process of the present invention in silicone oil
- Figure 5:: Microscopic image of form 1 with form 2 (about 15 %) obtained by the process of the present invention in silicone oil
- Figure 6:: Microscopic image of form 1 obtained by the prior art method in silicone oil

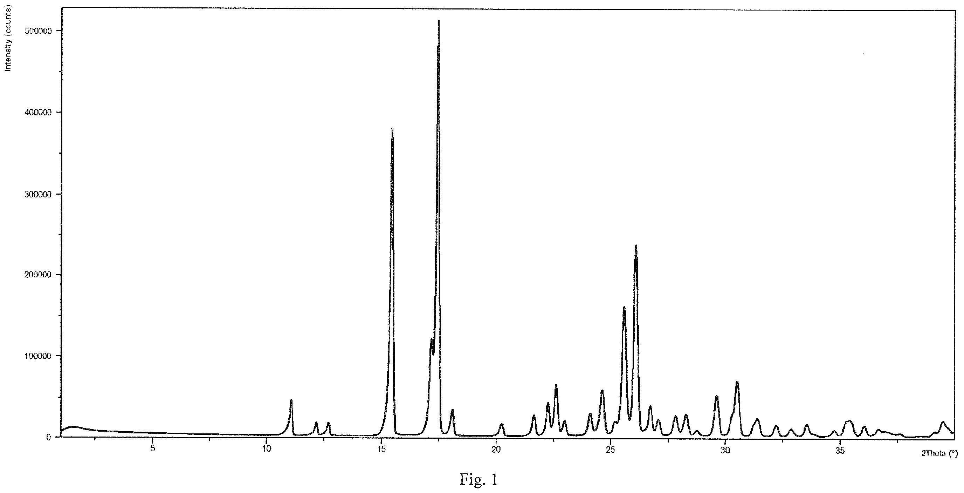

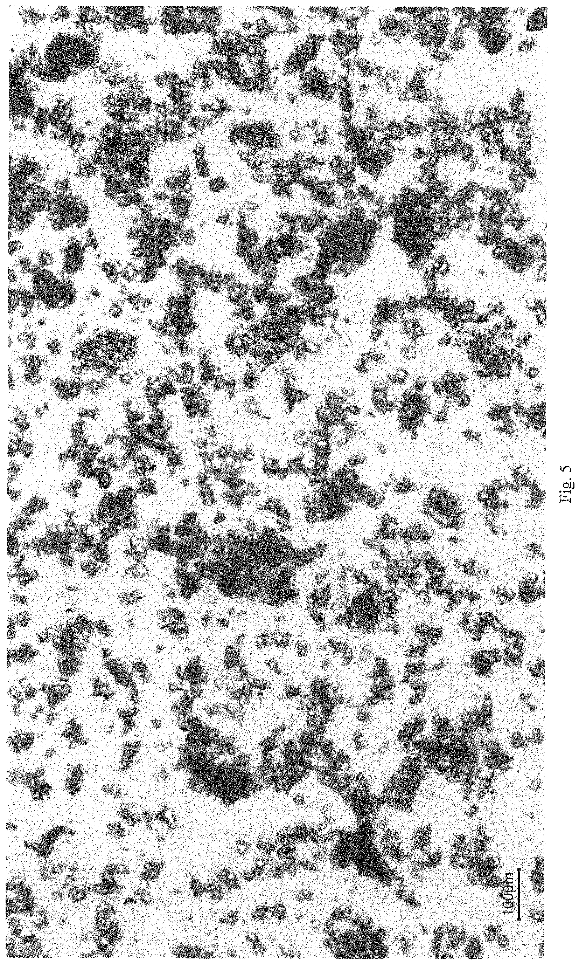

The polymorph form 1 of 3,4-DAPP is known from the prior art, for example the figure 1 of Powder Diffraction 2011, 26(4), 321-325, and figure 5 of Cryst. Growth Des. 2013, 13, 708-715, are showing powder XRD patterns of pure form 1 with essentially the same characteristic peaks as the pattern of the crystalline form 1 in the figure 1 of the current application. Also the simulated powder XRD patterns based on the single crystal data disclosed in these prior art publications are essentially the same as the pattern in the figure 1 of the present application.

The salt of the present invention is characterized by the following detailed list of peaks of the powder X-ray diffractogram of form 1 (Cu-Kα1 = 1.54059 Å): 11.06, 12.16, 12.69, 12.91, 15.45, 17.12, 17.42, 18.07, 20.21, 21.60, 22.22, 22.57, 22.95, 24.03, 24.59, 25.11, 25.54, 26.05, 26.69, 27.02, 27.76, 28.21, 28.69, 29.54, 30.19, 30.27 and 30.44 ± 0.20° 2θ. The polymorphically pure form 1 of amifampridine phosphate does not contain any additional peak in the range of 5.00 to 30.44 ± 0.20° 2θ. The complete powder XRD-pattern of form 1 is shown in table 1 below.

**Table 1. Form 1**

| **No.** | **Pos. [°2θ]** | **Relative Intensity [%]** |
|---|---|---|
| 1 | 11.0668(3) | 6.21 |
| 2 | 12.1645(9) | 2.23 |
| 3 | 12.6903(3) | 5.59 |
| 4 | 12.9147 | 0.52 |
| 5 | 15.4506(1) | 63.77 |
| 6 | 17.1273(2) | 22.46 |
| 7 | 17.4226(1) | 100 |
| 8 | 18.0740(5) | 7.22 |
| 9 | 20.2130(7) | 3.24 |
| 10 | 21.6028(6) | 5.04 |
| 11 | 22.2150(6) | 6.84 |
| 12 | 22.5749(4) | 13.16 |
| 13 | 22.9486(6) | 6.98 |
| 14 | 24.0290(6) | 4.54 |
| 15 | 24.5867(3) | 17.17 |
| 16 | 25.113(1) | 2.35 |
| 17 | 25.5379(2) | 50.19 |
| 18 | 26.0493(1) | 75.88 |
| 19 | 26.6906(5) | 7.6 |
| 20 | 27.023(1) | 3.63 |
| 21 | 27.7553(4) | 7.48 |
| 22 | 28.2138(2) | 5.11 |
| 23 | 28.6898(4) | 2.03 |
| 24 | 29.5430(1) | 13.56 |
| 25 | 30.19(1) | 2.64 |
| 26 | 30.271(6) | 3.45 |
| 27 | 30.4422(4) | 18.46 |
| 28 | 31.184(2) | 2.34 |
| 29 | 31.352(1) | 3.97 |
| 30 | 32.1460(2) | 3.74 |
| 31 | 32.78(4) | 1.42 |
| 32 | 32.88(7) | 0.85 |
| 33 | 33.4885(3) | 3.33 |
| 34 | 33.784(1) | 0.79 |
| 35 | 34.6712(4) | 1.84 |
| 36 | 35.15(2) | 2.6 |
| 37 | 35.27(1) | 2.71 |
| 38 | 35.430(6) | 3.19 |
| 39 | 35.9778(3) | 2.8 |
| 40 | 36.586(2) | 1.88 |
| 41 | 36.6640(8) | 0.68 |
| 42 | 36.932(7) | 1.02 |
| 43 | 37.33(3) | 0.06 |
| 44 | 37.531(1) | 0.93 |
| 45 | 39.050(1) | 1.08 |
| 46 | 39.4071(5) | 3.75 |
| 47 | 39.628(1) | 1.32 |

Polymorphically pure form 1 of amifampridine phosphate is further characterized by a PXRD pattern substantially the same as the pattern shown in Fig. 1.

Polymorphically pure form 1 is obtained if the phosphate salt of amifampridine is precipitated from an ethanolic solution containing amifampridine by adding an aqueous phosphoric acid solution. A mixture of the form 1 and the form 2 is obtained if amifampridine is dissolved in methanol, optionally in admixture with not more than 10 wt% water, and precipitating the phosphate salt of amifampridine by adding an aqueous phosphoric acid solution.

The complete powder XRD-pattern of form 1 with a minor amount of form 2 (about 15 %) is shown in table 2 below. Approximate estimate of 15% content of form 2 is based on the integral ratio of all peaks of known form 1 versus all peaks of form 2.

**Table 2. Form 1 with form 2**

| **No.** | **Pos. [°2θ]** | **Relative Intensity [%]** |
|---|---|---|
| 1 | 8.046(7) | 0.51 |
| 2 | 8.392(6) | 1.44 |
| 3 | 8.61(1) | 0.7 |
| 4 | 8.918(6) | 0.54 |
| 5 | 10.139(6) | 0.18 |
| 6 | 10.499(2) | 0.47 |
| 7 | 11.0814(3) | 7.54 |
| 8 | 12.1766(7) | 3.11 |
| 9 | 12.7021(7) | 3.4 |
| 10 | 12.941(6) | 0.32 |
| 11 | 13.129(8) | 0.34 |
| 12 | 13.478(1) | 0.2 |
| 13 | 14.908(2) | 0.19 |
| 14 | 15.4655(2) | 72.12 |
| 15 | 16.1589(7) | 2.19 |
| 16 | 17.1417(4) | 21.24 |
| 17 | 17.4436(2) | 100 |
| 18 | 18.0805(4) | 7.73 |
| 19 | 18.7404(8) | 0.86 |
| 20 | 19.0350(8) | 0.68 |
| 21 | 19.2946(5) | 1.17 |
| 22 | 19.6938(3) | 2.41 |
| 23 | 20.1998(2) | 3.22 |
| 24 | 21.1577(6) | 1.13 |
| 25 | 21.5871(2) | 5.22 |
| 26 | 22.2162(1) | 8.11 |
| 27 | 22.5725(1) | 13.24 |
| 28 | 22.9410(2) | 4.31 |
| 29 | 24.0655(2) | 5.83 |
| 30 | 24.5829(1) | 12.34 |
| 31 | 25.2020(8) | 2.88 |
| 32 | 25.53686(8) | 34.19 |
| 33 | 26.04124(5) | 56.84 |
| 34 | 26.6816(1) | 9.58 |
| 35 | 27.0425(2) | 4.19 |
| 36 | 27.7753(2) | 5.61 |
| 37 | 28.2320(2) | 5.45 |
| 38 | 28.7014(5) | 1.59 |
| 39 | 29.5568(1) | 11.24 |
| 40 | 30.260(1) | 4.95 |
| 41 | 30.4604(3) | 13.87 |
| 42 | 31.201(3) | 2.63 |
| 43 | 31.370(1) | 4.44 |
| 44 | 32.1541(3) | 2.73 |
| 45 | 32.778(3) | 0.76 |
| 46 | 32.876(1) | 1.2 |
| 47 | 33.5111(3) | 3.08 |
| 48 | 33.795(1) | 0.47 |

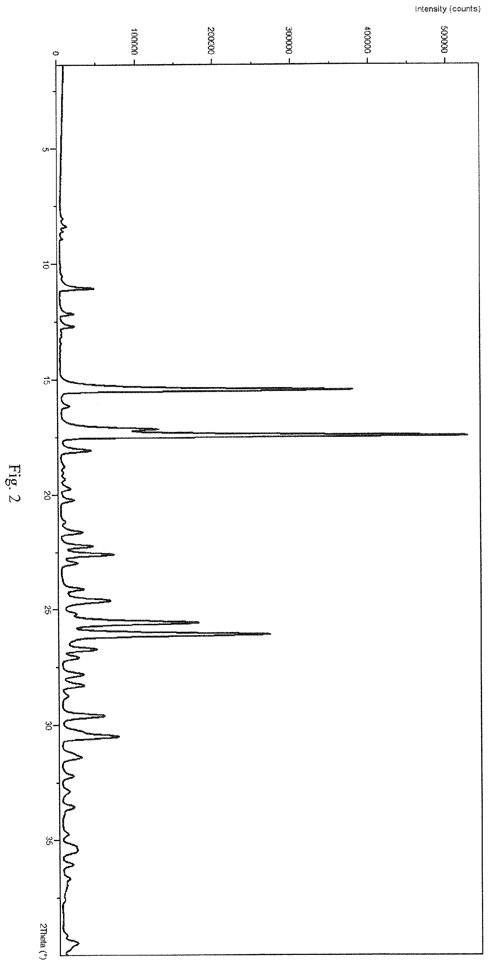

The comparison of table 1 with table 2 as well as the figure 1 with figure 2 leads to the conclusion that the most suitable peaks for the monitoring of the form 2 presence in the amifampridine phosphate API are the characteristic and non-interfering reflexes at 8.39, 16.16, 19.69, and 21.16 ± 0.2° 2θ.

In some embodiments, one or more additional reflexes at 8.05, 8.61, 8.92, 18.74, 19.29, and 21.16° ± 0.2° 2θ, which are also characteristic, non-interfering and well-visible peaks in the XRPD pattern despite signals from form 1, may be employed for the detection of the presence of crystalline form 2 as polymorphic impurity.

The powder X-ray diffraction pattern of the polymorphically pure form 1 of amifampridine phosphate API is showing only characteristic peaks of the polymorphic form 1, for example at 15.5, 17.4, 25.5 and 26.1 ± 0.2° 2θ, whilst no characteristic peaks are observed for form 2, for example at 8.39, 16.16, 19.69, and 21.16 ± 0.2° 2θ, and optionally one or more additional reflexes at 8.05, 8.61, 8.92, 18.74, 19.23 and 21.16° ± 0.2° 2θ.

The present invention further relates to a process for preparing a phosphate salt of amifampridine, wherein the process comprises the steps:
i) preparing an alcoholic solution containing amifampridine, wherein the solvent is an alcohol optionally in admixture with not more than 10 wt% water, wherein the alcohol is selected from methanol, ethanol, n-propanol and iso-propanol,
ii) precipitating the phosphate salt of amifampridine by adding an aqueous phosphoric acid solution to the alcoholic solution obtained in step (i), wherein the aqueous phosphoric acid solution contains at least 70 wt% phosphoric acid, and
iii) isolating the phosphate salt of amifampridine obtained in step (ii).

Preferably, the solvent is ethanol optionally in admixture with not more than 10 wt% water. In a preferred embodiment, the solvent is ethanol in admixture with not more than 5 wt% water.

The addition of the aqueous phosphoric acid solution to the alcoholic solution is preferably conducted at elevated temperature, e.g. at 50-70°C if an ethanolic solution is used (cf. Example 1). Heating the alcoholic solution allows the preparation of much higher concentrated solutions and, thus, saves solvent and, furthermore, allows to prepare larger batch sizes of the active drug ingredient in the same reactor.

Preferably, the aqueous phosphoric acid solution contains at least 80 wt%, more preferably at least 85 wt% phosphoric acid.

The phosphate salt of amifampridine obtained by the process of the present invention can be directly processed into a solid unit dosage form for oral administration, i.e. without further purification and without micronization. Hence, the present invention also relates to a process for preparing a solid unit dosage form for oral administration containing a phosphate salt of amifampridine, wherein the process comprises the steps:
i) preparing an alcoholic solution containing amifampridine, wherein the solvent is an alcohol optionally in admixture with not more than 10 wt% water, wherein the alcohol is selected from methanol, ethanol, n-propanol and iso-propanol,
ii) precipitating the phosphate salt of amifampridine by adding an aqueous phosphoric acid solution to the alcoholic solution obtained in step (i), wherein the aqueous phosphoric acid solution contains at least 70 wt% phosphoric acid,
iii) isolating the phosphate salt of amifampridine obtained in step (ii),
iv) mixing the phosphate salt of amifampridine obtained in step (iii) and a pharmaceutical excipient to obtain a powdery blend, and
v) converting the powdery blend obtained in step (iv) into the solid unit dosage form.

Preferably, the solvent is ethanol optionally in admixture with not more than 10 wt% water. In a preferred embodiment, the solvent is ethanol in admixture with not more than 5 wt% water.

It is also preferred that the aqueous phosphoric acid solution contains at least 80 wt%, more preferred at least 85 wt% phosphoric acid.

The solid unit dosage form may be an optionally film-coated tablet. The tablet may be prepared by a process, wherein step (v) comprises
a) subjecting the powdery blend obtained in step (iv) to compression to obtain the tablet, or
b1) subjecting the powdery blend obtained in step (iv) to compaction,
b2) milling the compacted material obtained in step (b1) to obtain granules,
b3) optionally mixing the granules obtained in step (b2) and a pharmaceutical excipient to obtain a mixture, and
b4) subjecting the granules obtained in step (b2) or the mixture obtained in step (b3) to compression to obtain the tablet.

Hence, the phosphate salt of amifampridine of the present invention can be used in the preparation of a tablet through direct compression or dry-granulation, whereby direct compression (above method (a)) is preferred. The phosphate salt of amifampridine of the present invention can be used for the treatment of botulism, myasthenia gravis, congenital myasthenic syndromes (CMS), Lambert-Eaton myasthenic syndrome (LEMS) or fatigue related to a neurological pathology, such as multiple sclerosis and amyotrophic lateral sclerosis (ALS). The invention is further illustrated by reference to the following examples.

### Examples

### Analytical methods:

### Appearance:

The appearance of the drug substance is determined by visual inspection.

### ¹H-NMR:

The drug substance (about 20 mg) is weighed, dissolved in 0.6 mL deuterium oxide (D₂O) and measured using Bruker 500 MHz Avance^{™} III HD NMR spectrometer.

### Melting Point:

The melting point (m.p.) is measured automatically by a digital melting point system Mettler Toledo MP70 according to Ph. Eur 2.2.14.

### DSC:

DSC data are obtained on Netzsch Phoenix DSC 204 F1. The drug substance sample (about 10 mg) is placed into a DSC pan and weight accurately recorded. Sealed aluminum pan with one pinhole is used for analysis. The sample is heated under nitrogen atmosphere at a rate of 10°C/min.

### Related substances by HPLC:

### Chemicals:

Water (H₂O); HPLC gradient grade
Acetonitrile (ACN); HPLC gradient grade
Trifluoroacetic acid (TFA)

### Chromatographic system:

| | |
|---|---|
| Column: | Phenomenex: Gemini C6-Hexyl, 250 x 4,6 mm, 110 Å |
| Mobile phase: | Eluent A: ACN:H₂O (10:90) 0.1 % TFA |
| Flowrate: | 0.8 mL/min |
| Column temperature: | 25°C |
| Detector: | UV 230 nm |
| Inject volume: | 20 µL |
| Mode | Isocratic |
| Acquisition time | 15 min |

*Preparation of sample (test solution):* The drug substance (about 10 mg) is weighed in a 25 mL volumetric flask, dissolved in acetonitrile and the flask is filled to the mark with acetonitrile.

### XRPD:

The qualitative PXRD measurements were performed by cGMP-accredited XRPD laboratory DANNALAB B.V. (Enschede, the Netherlands) using X'Pert MPD system.

A summary of XRPD method parameters is described below:
PANalytical X'Pert MPD equipped with LLF x-ray source with Cu-Kα1 radiation, Soller collimators of 0.02, 0.04 rad, divergent slit ½ deg and focusing X-Ray mirror are used. The powder load was 400 mg. The radiation used is Cu-Kα1 = 1.54059 Å (8keV); 40kV-40mA; measurement range 2-40 degrees; measurement time per scan - 2.5 hours; step = 0.013° 2θ. During the measurements the sample was rotated at speed 4s/revolution to improve counting statistic.

### Sample preparation instruction:

Prepare the sample using PANalytical sample preparation instruction for loading sample in transmission sample holder. Place the prepared sample holder on the sample changer stage of the XRD instrument and analyze as per the above method at room temperature.

### Particle size measurement (PSD):

The particle size determination was done using Malvern Mastersizer 2000 and following dry method using 1 g of the sample:

### Typical conditions are:

| | | |
|---|---|---|
| 1. | Particle RI: | 1.00 |
| 2. | Obscuration range: | 1-6 %, |
| 3. | Air pressure: | 2 bar |
| 4. | Vibration feed rate: | 50 % |
| 5. | Measurement time: | 6 seconds |
| 6. | Background time: | 6 seconds |
| 7. | Measurement: | 1 per aliquot |
| 8. | No of Aliquots: | 1 per SOP |

### Microscopy:

Microscopic images were taken by contract laboratory solid-chem GmbH (Bochum, Germany) with the help of a Leica DMRB microscope. The samples were placed, in pure silicone oil, on a microscope slide and examined, under polarizable light, regarding the crystallite size and habit.

### Comparative example

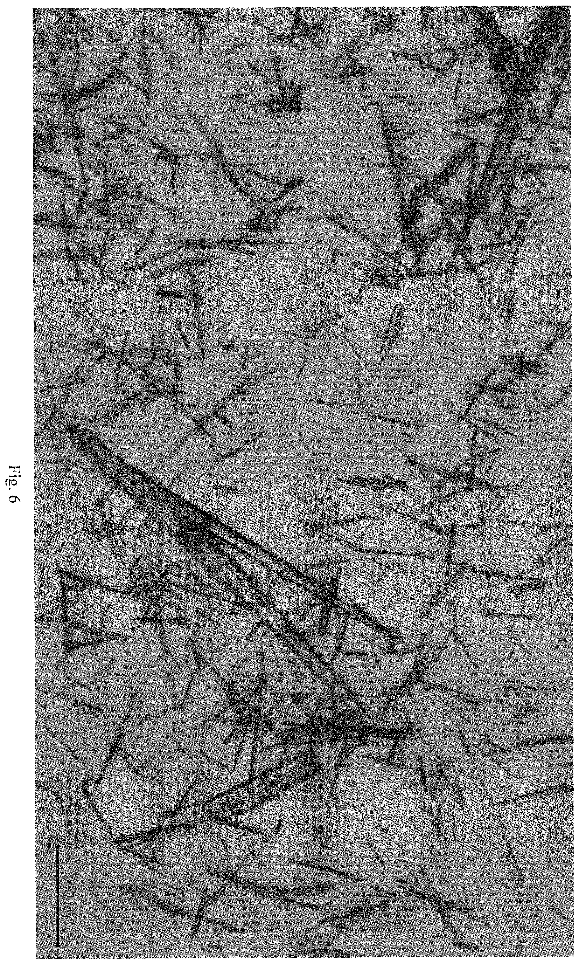

50 g amifampridine were dissolved in 1100 ml water by heating the suspension to 80°C. Subsequently, 2 equivalents phosphoric acid (85 wt%) were added. After cooling (ambient temperature) 600 ml water were removed under reduced pressure and 583 ml ethanol were added slowly to the concentrated aqueous solution to initiate the crystallization of the product. The reaction mixture was kept at ambient temperature overnight without stirring. The crystals were isolated by filtration, washed with ethanol and dried under reduced pressure at 60°C to yield 61 g (64%) of the crystalline form 1 of amifampridine phosphate as a beige, voluminous, cotton-like material (figure 6). Related substances by HPLC: unknown impurity-1: 0.178%, unknown Impurity-2: 0.202%, total impurities: 0.38%.

### Example 1

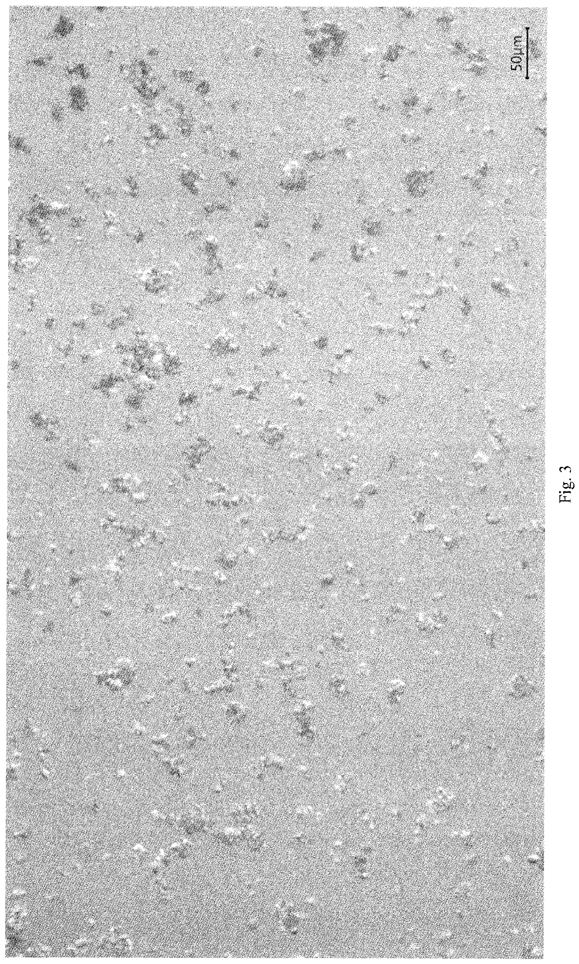

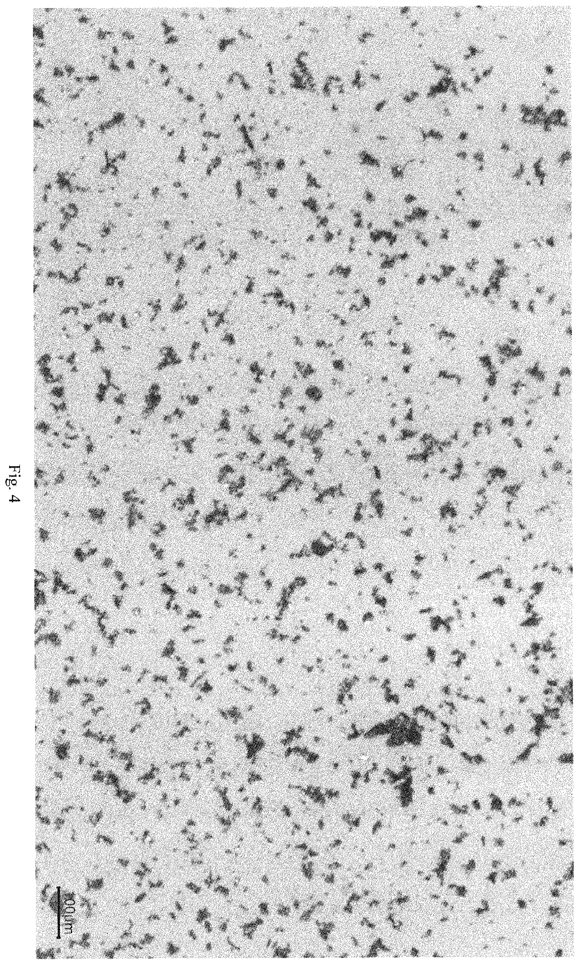

In a 30 L reactor, 300.03 g (2.75 mol) amifampridine were dissolved in 6.0 L ethanol under mechanical stirring at 133 rpm and under heating to Tᵢ = 68.4°C. The solution was allowed to cool to Tᵢ = 54.3°C. Subsequently, 349.70 g (3.02 mol; 1.1 eq.) phosphoric acid 85 % were added within 24 min (T_{i,max} = 60.4°C). The product precipitated immediately upon addition of the acid, and the resulting suspension was stirred for 17 h 8 min at Tᵢ = 22.0°C. Subsequently, the suspension was suction filtered and washed twice with each 1.0 L ethanol and twice with each 1.0 L methyl tert-butyl ether (MTBE). The product was dried under reduced pressure (17 mbar) at T_{bath} = 60°C to yield 568.08 g (2.74 mol; 99.7 %) amifampridine phosphate as a white, free flowing solid (irregular shape, mainly flakes; figures 3 and 4).

**¹H-NMR** (D₂O, 500 MHz): δ = 7.64 (dd, 1 H, *³J₁* = 6.6 Hz; *³J₂* = 1.2 Hz), 7.65 (d, 1 H, *³J* = 1.2 Hz), 6.78 (d, 1 H, *³J* = 6.6 Hz).

**m.p.:** 225.0-230.4°C; **DSC:** onset: 228.06°C, peak: 231.37°C, endset: 236.62 °C **XRPD:** form 1 (figure 1).

**PSD by Malvern (by volume):** D₉₀: 15.028 µm, D₅₀: 4.396 µm, D₁₀: 0.474 µm. **Related substances by HPLC:** unknown impurity-1: not detected, unknown impurity-2: not detected, total impurities: not detected.

### Example 2

7.01 g amifampridine were dissolved in 105 ml methanol at 30°C under mechanical stirring. The solution was allowed to cool to 19.5°C. Subsequently, phosphoric acid (85 wt%) was added dropwise within 15 min under stirring. A precipitate appears immediately upon addition. After the addition of phosphoric acid, the obtained suspension was stirred for 15 min and subsequently cooled to 2°C and stirred for 1 h. The precipitate was isolated by filtration and washed with methanol. The product was dried at Tbath = 60°C under reduced pressure (19 mbar), which yields form 1 with about 15 % form 2 (figure 2) as a white, free-flowing solid (plate-like shape; figure 5).

## Claims

1. Phosphate salt of amifampridine, wherein the salt is **characterized by** the following peaks of the powder X-ray diffractogram (Cu-Kα1 = 1.54059 Å): 11.06, 12.16, 12.69, 12.91, 15.45, 17.12, 17.42, 18.07, 20.21, 21.60, 22.22, 22.57, 22.95, 24.03, 24.59, 25.11, 25.54, 26.05, 26.69, 27.02, 27.76, 28.21, 28.69, 29.54, 30.19, 30.27 and 30.44 ± 0.20° 2θ, and wherein the salt is obtainable by a process consisting of the steps:
i) preparing an alcoholic solution containing amifampridine, wherein the solvent is an alcohol optionally in admixture with not more than 10 wt% water, wherein the alcohol is selected from methanol, ethanol, n-propanol and iso-propanol,
ii) precipitating the phosphate salt of amifampridine by adding an aqueous phosphoric acid solution to the alcoholic solution obtained in step (i), wherein the aqueous phosphoric acid solution contains at least 70 wt% phosphoric acid, and
iii) isolating the phosphate salt of amifampridine obtained in step (ii).

2. The salt according to claim 1, wherein the powder X-ray diffractogram does not contain an additional peak in the range of 5.00 to 30.44 ± 0.20° 2θ.

3. The salt according to claim 1, wherein the powder X-ray diffractogram does not contain peaks at 8.39, 16.16, 19.69 and 21.16 ± 0.20° 2θ.

4. A process for preparing a phosphate salt of amifampridine, wherein the process consists of the steps:
i) preparing an alcoholic solution containing amifampridine, wherein the solvent is an alcohol optionally in admixture with not more than 10 wt% water, wherein the alcohol is selected from methanol, ethanol, n-propanol and iso-propanol,
ii) precipitating the phosphate salt of amifampridine by adding an aqueous phosphoric acid solution to the alcoholic solution obtained in step (i), wherein the aqueous phosphoric acid solution contains at least 70 wt% phosphoric acid, and
iii) isolating the phosphate salt of amifampridine obtained in step (ii).

5. The process according to claim 4, wherein the solvent is ethanol optionally in admixture with not more than 10 wt% water.

6. The process according to claim 5, wherein the solvent is ethanol in admixture with not more than 5 wt% water.

7. The process according to any one of claims 4 to 6, wherein the aqueous phosphoric acid solution contains at least 80 wt%, preferably at least 85 wt% phosphoric acid.

8. A process for preparing a solid unit dosage form for oral administration containing a phosphate salt of amifampridine, wherein the process consists of the steps:
i) preparing an alcoholic solution containing amifampridine, wherein the solvent is an alcohol optionally in admixture with not more than 10 wt% water, wherein the alcohol is selected from methanol, ethanol, n-propanol and iso-propanol,
ii) precipitating the phosphate salt of amifampridine by adding an aqueous phosphoric acid solution to the alcoholic solution obtained in step (i), wherein the aqueous phosphoric acid solution contains at least 70 wt% phosphoric acid,
iii) isolating the phosphate salt of amifampridine obtained in step (ii),
iv) mixing the phosphate salt of amifampridine obtained in step (iii) and a pharmaceutical excipient to obtain a powdery blend, and
v) converting the powdery blend obtained in step (iv) into the solid unit dosage form.

9. The process according to claim 8, wherein the solvent is ethanol optionally in admixture with not more than 10 wt% water.

10. The process according to claim 9, wherein the solvent is ethanol in admixture with not more than 5 wt% water.

11. The process according to any one of claims 8 to 10, wherein the aqueous phosphoric acid solution contains at least 80 wt%, preferably at least 85 wt% phosphoric acid.

12. The process according to any one of claims 8 to 11, wherein the solid unit dosage form is an optionally film-coated tablet.

13. The process according to claim 12, wherein step (v) comprises
a) subjecting the powdery blend obtained in step (iv) to compression to obtain the tablet, or
b1) subjecting the powdery blend obtained in step (iv) to compaction,
b2) milling the compacted material obtained in step (b1) to obtain granules,
b3) optionally mixing the granules obtained in step (b2) and a pharmaceutical excipient to obtain a mixture, and
b4) subjecting the granules obtained in step (b2) or the mixture obtained in step (b3) to compression to obtain the tablet.

## Patentansprüche

1. Phosphatsalz von Amifampridin, wobei das Salz durch die folgenden Signale im Röntgen-Pulverdiffraktogramm (Cu-Kα1 = 1.54059 Å) gekennzeichnet ist: 11.06, 12.16, 12.69, 12.91, 15.45, 17.12, 17.42, 18.07, 20.21, 21.60, 22.22, 22.57, 22.95, 24.03, 24.59, 25.11, 25.54, 26.05, 26.69, 27.02, 27.76, 28.21, 28.69, 29.54, 30.19, 30.27 und 30.44 ± 0.20° 2θ, und wobei das Salz durch ein Verfahren erhältlich ist, das aus folgenden Schritten besteht:
i) Herstellen einer alkoholischen Amifampridin enthaltenden Lösung, wobei das Lösungsmittel ein Alkohol ist, gegebenenfalls im Gemisch mit nicht mehr als 10 Gew.-% Wasser, wobei der Alkohol aus Methanol, Ethanol, n-Propanol und Isopropanol ausgewählt ist,
ii) Fällen des Phosphatsalzes von Amifampridin durch Zugabe einer wässrigen Phosphorsäurelösung zu der im Schritt (i) erhaltenen alkoholischen Lösung, wobei die wässrige Phosphorsäurelösung mindestens 70 Gew.-% Phosphorsäure enthält,
iii) Isolieren des im Schritt (ii) erhaltenen Phosphatsalzes von Amifampridin.

2. Salz gemäß Anspruch 1, wobei das Röntgen-Pulverdiffraktogramm kein zusätzliches Signal im Bereich von 5.00 bis 30.44 ± 0.20° 2θ aufweist.

3. Salz gemäß Anspruch 1, wobei das Röntgen-Pulverdiffraktogramm keine Signale bei 8.39, 16.16, 19.69 und 21.16 ± 0.20° 2θ aufweist.

4. Verfahren zur Herstellung eines Phosphatsalzes von Amifampridin, wobei das Verfahren aus den Schritten besteht:
i) Herstellen einer alkoholischen Amifampridin enthaltenden Lösung, wobei das Lösungsmittel ein Alkohol ist, gegebenenfalls im Gemisch mit nicht mehr als 10 Gew.-% Wasser, wobei der Alkohol aus Methanol, Ethanol, n-Propanol und Isopropanol ausgewählt ist,
ii) Fällen des Phosphatsalzes von Amifampridin durch Zugabe einer wässrigen Phosphorsäurelösung zu der im Schritt (i) erhaltenen alkoholischen Lösung, wobei die wässrige Phosphorsäurelösung mindestens 70 Gew.-% Phosphorsäure enthält,
iii) Isolieren des im Schritt (ii) erhaltenen Phosphatsalzes von Amifampridin.

5. Verfahren gemäß Anspruch 4, wobei das Lösungsmittel Ethanol ist, gegebenenfalls im Gemisch mit nicht mehr als 10 Gew.-% Wasser.

6. Verfahren gemäß Anspruch 5, wobei das Lösungsmittel Ethanol im Gemisch mit nicht mehr als 5 Gew.-% Wasser ist.

7. Verfahren gemäß einem der Ansprüche 4 bis 6, wobei die wässrige Phosphorsäurelösung mindestens 80 Gew.-%, vorzugsweise mindestens 85 Gew.-% Phosphorsäure enthält.

8. Verfahren zur Herstellung einer festen Darreichungsform für die orale Verabreichung enthaltend ein Phosphatsalz von Amifampridin, wobei das Verfahren aus den Schritten besteht:
i) Herstellen einer alkoholischen Amifampridin enthaltenden Lösung, wobei das Lösungsmittel ein Alkohol ist, gegebenenfalls im Gemisch mit nicht mehr als 10 Gew.-% Wasser, wobei der Alkohol aus Methanol, Ethanol, n-Propanol und Isopropanol ausgewählt ist,
ii) Fällen des Phosphatsalzes von Amifampridin durch Zugabe einer wässrigen Phosphorsäurelösung zu der im Schritt (i) erhaltenen alkoholischen Lösung, wobei die wässrige Phosphorsäurelösung mindestens 70 Gew.-% Phosphorsäure enthält,
iii) Isolieren des im Schritt (ii) erhaltenen Phosphatsalzes von Amifampridin,
iv) Mischen des im Schritt (iii) erhaltenen Phosphatsalzes von Amifampridin mit einem pharmazeutischen Hilfsstoff, um eine Pulvermischung zu erhalten,
v) Überführen der im Schritt (iv) erhaltenen Pulvermischung in eine feste Darreichungsform.

9. Verfahren gemäß Anspruch 8, wobei das Lösungsmittel Ethanol ist, gegebenenfalls im Gemisch mit nicht mehr als 10 Gew.-% Wasser.

10. Verfahren gemäß Anspruch 9, wobei das Lösungsmittel Ethanol im Gemisch mit nicht mehr als 5 Gew.-% Wasser ist.

11. Verfahren gemäß einem der Ansprüche 8 bis 10, wobei die wässrige Phosphorsäurelösung mindestens 80 Gew.-%, vorzugsweise 85 Gew.-% Phosphorsäure enthält.

12. Verfahren gemäß einem der Ansprüche 8 bis 11, wobei die feste Darreichungsform eine gegebenenfalls filmbeschichtete Tablette ist.

13. Verfahren gemäß Anspruch 12, wobei Schritt (v) umfasst:
a) Verpressen der im Schritt (iv) erhaltenen Pulvermischung, um eine Tablette zu erhalten, oder
b1) Kompaktieren der im Schritt (iv) erhaltenen Pulvermischung,
b2) Malen des im Schritt (b1) erhaltenen kompaktierten Materials, um ein Granulat zu erhalten,
b3) gegebenenfalls Mischen des im Schritt (b2) erhaltenen Granulats mit einem pharmazeutischen Hilfsstoff, um ein Gemisch zu erhalten,
b4) Verpressen des im Schritt (b2) erhaltenen Granulats oder des im Schritt (b3) erhaltenen Gemischs, um die Tablette zu erhalten.

## Revendications

1. Sel de phosphate d'amifampridine, dans lequel le sel est **caractérisé par** les pics suivants du diffractogramme de rayons X sur poudre (Cu-Kα1 = 1,54059 Å) : 11,06, 12,16, 12,69, 12,91, 15,45, 17,12, 17,42, 18,07, 20,21, 21,60, 22,22, 22,57, 22,95, 24,03, 24,59, 25,11, 25,54, 26,05, 26,69, 27,02, 27,76, 28,21, 28,69, 29,54, 30,19, 30,27 et 30,44 ± 0,20°2θ, et dans lequel le sel peut être obtenu par un procédé comprenant les étapes consistant à :
i) préparer une solution alcoolique contenant de l'amifampridine, dans lequel le solvant est un alcool facultativement en mélange avec pas plus de 10 % en poids d'eau, dans lequel l'alcool est choisi parmi le méthanol, l'éthanol, le n-propanol et l'isopropanol,
ii) précipiter le sel de phosphate d'amifampridine en ajoutant une solution aqueuse d'acide phosphorique à la solution alcoolique obtenue à l'étape (i), dans lequel la solution aqueuse d'acide phosphorique contient au moins 70 % en poids d'acide phosphorique, et
iii) isoler le sel de phosphate d'amifampridine obtenu à l'étape (ii).

2. Sel selon la revendication 1, dans lequel le diffractogramme de rayons X sur poudre ne contient pas de pic supplémentaire dans la plage de 5,00 à 30,44 ± 0,20°2θ.

3. Sel selon la revendication 1, dans lequel le diffractogramme de rayons X sur poudre ne contient pas de pics à 8,39, 16,16, 19,69 et 21,16 ± 0,20°2θ.

4. Procédé de préparation d'un sel de phosphate d'amifampridine, dans lequel le procédé comprend les étapes consistant à :
i) préparer une solution alcoolique contenant de l'amifampridine, dans lequel le solvant est un alcool facultativement en mélange avec pas plus de 10 % en poids d'eau, dans lequel l'alcool est choisi parmi le méthanol, l'éthanol, le n-propanol et l'isopropanol,
ii) précipiter le sel de phosphate d'amifampridine en ajoutant une solution aqueuse d'acide phosphorique à la solution alcoolique obtenue à l'étape (i), dans lequel la solution aqueuse d'acide phosphorique contient au moins 70 % en poids d'acide phosphorique, et
iii) isoler le sel de phosphate d'amifampridine obtenu à l'étape (ii).

5. Procédé selon la revendication 4, dans lequel le solvant est facultativement mélangé avec pas plus de 10 % en poids d'eau.

6. Procédé selon la revendication 5, dans lequel le solvant est de l'éthanol en mélange avec pas plus de 5 % en poids d'eau.

7. Procédé selon l'une quelconque des revendications 4 à 6, dans lequel la solution aqueuse d'acide phosphorique contient au moins 80 % en poids, de préférence au moins 85 % en poids d'acide phosphorique.

8. Procédé de préparation d'une forme posologique unitaire solide pour administration orale contenant un sel de phosphate d'amifampridine, dans lequel le procédé comprend les étapes consistant à :
i) préparer une solution alcoolique contenant de l'amifampridine, dans lequel le solvant est un alcool facultativement en mélange avec pas plus de 10 % en poids d'eau, dans lequel l'alcool est choisi parmi le méthanol, l'éthanol, le n-propanol et l'isopropanol,
ii) précipiter le sel de phosphate d'amifampridine en ajoutant une solution aqueuse d'acide phosphorique à la solution alcoolique obtenue à l'étape (i), dans lequel la solution aqueuse d'acide phosphorique contient au moins 70 % en poids d'acide phosphorique,
iii) isoler le sel de phosphate d'amifampridine obtenu à l'étape (ii),
iv) mélanger le sel de phosphate d'amifampridine obtenu à l'étape (iii) et un excipient pharmaceutique pour obtenir un mélange pulvérulent, et
v) convertir le mélange pulvérulent obtenu à l'étape (iv) en une forme posologique unitaire solide.

9. Procédé selon la revendication 8, dans lequel le solvant est de l'éthanol facultativement en mélange avec pas plus de 10 % en poids d'eau.

10. Procédé selon la revendication 9, dans lequel le solvant est de l'éthanol en mélange avec pas plus de 5 % en poids d'eau.

11. Procédé selon l'une quelconque des revendications 8 à 10, dans lequel la solution aqueuse d'acide phosphorique contient au moins 80 % en poids, de préférence au moins 85 % en poids d'acide phosphorique.

12. Procédé selon l'une quelconque des revendications 8 à 11, dans lequel la forme posologique unitaire solide est un comprimé facultativement pelliculé.

13. Procédé selon la revendication 12, dans lequel l'étape (v) comprend les étapes consistant à
a) soumettre le mélange pulvérulent obtenu à l'étape (iv) à une compression pour obtenir le comprimé, ou
b1) soumettre le mélange pulvérulent obtenu à l'étape (iv) à un compactage,
b2) broyer le matériau compacté obtenu à l'étape (b1) pour obtenir des granulés,
b3) mélanger facultativement les granulés obtenus à l'étape (b2) et un excipient pharmaceutique pour obtenir un mélange, et
b4) soumettre les granulés obtenus à l'étape (b2) ou le mélange obtenu à l'étape (b3) à une compression pour obtenir le comprimé.
